# EUROPEAN PATENT SPECIFICATION

(11) **EP 3 117 757 B1**
(45) Date of publication and mention of the grant of the patent: **19.02.2020**
(21) Application number: 15762128.5
(22) Date of filing: 15.01.2015
(51) Int. Cl.: A61B 1/00, A61B 1/04, A61B 1/045, A61B 1/05, A61B 1/06, A61B 5/1455, A61B 5/1459, G02B 23/24, G02B 23/26, H04N 5/225, A61B 5/00, H04N 7/18

(54) **ENDOSCOPE SYSTEM**
ENDOSKOPSYSTEM
SYSTÈME D'ENDOSCOPE

(30) Priority: 12.03.2014 JP 2014048895
(43) Date of publication of application: 18.01.2017
(73) Proprietor: FUJIFILM Corporation, Tokyo 106-8620 (JP)
(72) Inventor: YANO Takashi, Ashigarakami-gun Kanagawa 258-8538 (JP); MURAYAMA Jin, Ashigarakami-gun Kanagawa 258-8538 (JP); SETO Hideo, Ashigarakami-gun Kanagawa 258-8538 (JP); KOSHIBA Masaaki, Ashigarakami-gun Kanagawa 258-8538 (JP)
(74) Representative: Klunker IP Patentanwälte PartG mbB
(86) International application number: PCT/JP2015/050869
(87) International publication number: WO 2015/136963

(56) References cited:
- EP-A1- 2 664 268
- JP-A- 2007 166 486
- JP-A- 2010 172 673
- JP-A- 2010 184 047
- JP-A- 2011 250 926
- JP-A- 2012 019 982
- JP-A- 2012 023 492
- JP-A- 2012 253 624
- JP-A- 2013 165 776
- JP-B1- 5 245 022
- JP-B2- 5 259 882
- JP-B2- 5 274 720
- JP-B2- 5 296 930
- JP-B2- 5 435 916
- US-A- 6 124 888
- US-A1- 2010 069 713
- US-A1- 2013 211 217

## Description

### BACKGROUND OF THE INVENTION

### 1. Field of the Invention

The present invention relates to an endoscope system for obtaining biological function information relating to the oxygen saturation level of blood hemoglobin from image capture signals obtained by capturing images of an observation region in a living body.

### 2. Description of the Related Art

In the field of medicine, diagnoses and so on using an endoscope system that includes a light source apparatus, an endoscope, and a processor apparatus are widely made. The endoscope system has a normal observation mode in which normal white light is emitted to an observation region in a living body and observation is performed, and a special observation mode in which special light is emitted to an observation region and observation is performed. As the special observation mode, a mode in which the oxygen saturation level of blood hemoglobin in the observation region can be obtained is known (see Japanese Unexamined Patent Application Publication No. 2012-213550). The oxygen saturation level is biological function information with which normal tissue and cancerous tissue can be distinguished from each other.

In the special observation mode, first illumination light and second illumination light are alternately supplied to the endoscope from the light source apparatus and are emitted to the observation region from the distal end section of the endoscope. The first illumination light is normal light. The second illumination light is special light having a spectral property different from that of the first illumination light and including different-absorption-wavelength light that has different absorption coefficients for oxygenated hemoglobin and reduced hemoglobin. The processor apparatus calculates the oxygen saturation level on the basis of image capture signals obtained by using the first and second illumination light and generates an oxygen saturation image (special observation image). The processor apparatus also generates a normal observation image on the basis of an image capture signal obtained by capturing with an image sensor an image of the observation region to which the first illumination light is emitted.

In a conventional endoscope system, a CCD (Charge Coupled Device) image sensor is used as an image sensor of the endoscope. However, a CMOS (Complementary Metal-Oxide Semiconductor) image sensor has been increasingly used in recent years (see JP5249882B2 and JP2010-68992A = US 2010/069713 A1). This is because a CMOS image sensor has lower power consumption than a CCD image sensor, and peripheral circuits, such as an ADC (analog-to-digital converter) circuit, can be formed on the same substrate on which an image capture unit is formed. The CMOS image sensor basically employs a rolling shutter method in which resetting and signal reading are performed for a plurality of pixel rows formed on the image capture unit one by one sequentially. The period from resetting to signal reading for each pixel row is the exposure period.

In the rolling shutter method, the exposure timing for each of the pixel rows shifts one by one sequentially. Therefore, when the illumination light is switched from normal light to special light without interruption while the image sensor is driven in accordance with the rolling shutter method, the exposure periods of some pixel rows extend beyond the switching of the illumination light, and an image of light in which the normal light and the special light mix is captured (see Japanese Unexamined Patent Application Publication No. 2010-68992). Therefore, Japanese Unexamined Patent Application Publication No. 2010-68992 proposes a technique in which a turn-off period is provided upon switching between the normal light and the special light and signal reading is performed during the turn-off period.

US 2013/0211217 A1 discloses a processor apparatus for an endoscope in which oxygen saturation images are generated based on a first and a second image so as to visualize potential cancerous regions in endoscopic images. When an exposure time of a first illumination light is equal to an exposure time of a second illumination light, also a first read time and a second read time are equal.

### SUMMARY OF THE INVENTION

If a turn-off period is simply provided upon switching between the normal light and the special light as described above, the frame rate of the image sensor decreases due to the provided turn-off period. Therefore, in Japanese Unexamined Patent Application Publication No. 2010-68992, the emission time (exposure time) of the normal light and that of the special light are reduced, and the read time is reduced by decreasing the number of pixels of the image sensor from which signal reading is performed to thereby prevent the frame rate from decreasing.

As described above, in the special observation mode described in JP2010-68992A, the exposure time is reduced and the pixels are skipped, which results in a decrease in the brightness and resolution of both the normal observation image and the special observation image. The special observation mode described in JP2010-68992A is a mode in which narrowband light is used as the special light and a specific tissue, a lesion, and so on are highlighted. A diagnosis is made by comparing the normal observation image with the special observation image obtained in the special observation mode. Therefore, even if the brightness and resolution of both of the images decrease, no special problem arises as long as the brightness and resolution of both of the images are the same.

However, in the special observation mode in which the oxygen saturation level of blood hemoglobin is obtained, a diagnosis is made by using an oxygen saturation image (an image in which the color of a hypoxic portion is changed to blue, for example) that is generated by performing, on the basis of the oxygen saturation level, image processing on a normal observation image obtained as a result of emission of the first illumination light during the special observation mode. The oxygen saturation image is an image based on the normal observation image as described above, and therefore, it is desirable that a decrease in the brightness and resolution does not occur compared to a normal observation image obtained in the normal observation mode and that the appearance remains unchanged.

An object of the present invention is to provide an endoscope system with which a decrease in the frame rate can be suppressed without a decrease in the brightness and resolution of an oxygen saturation image.

In order to accomplish the above-described object, an endoscope system according to the present invention includes the features of claim 1. Preferred embodiments are defined by the dependent claims. Any example or embodiment which does not fall under the scope of the independent claim is not part of the invention.

Preferably, the controller drives the image sensor in the normal observation mode in accordance with a rolling shutter method in which each row of the pixels is sequentially selected in the column direction and signal reading and resetting are performed, and drives the image sensor in the special observation mode in accordance with a global shutter method in which the pixels are collectively reset in association with a start of emission of each of the first illumination light and the second illumination light, the pixels are sequentially selected in the column direction, and signal reading is performed after emission of each of the first illumination light and the second illumination light is stopped.

Preferably, the plurality of pixels are divided into a plurality of types of pixel groups having different spectral sensitivities, and the controller reads only signals from a pixel group of highest sensitivity to the different-absorption-wavelength light as the second image capture signal to thereby make the second read time shorter than the first read time.

The controller may increase a clock frequency of a control signal applied to the image sensor to thereby make the second read time shorter than the first read time.

The controller may drive the image sensor in accordance with a pixel addition method in the column direction to thereby make the second read time shorter than the first read time.

The controller may reduce a pixel area of the image sensor from which signals are read to thereby make the second read time shorter than the first read time.

The image sensor may include a column ADC circuit in which an ADC that converts an analog signal into a digital signal is arranged for each column of the pixels, and the controller may increase a temporal change rate of a reference signal of the column ADC circuit to thereby make the second read time shorter than the first read time.

The controller may reduce a blanking interval of the image sensor to thereby make the second read time shorter than the first read time.

The controller may make an emission time of the second illumination light shorter than an emission time of the first illumination light in the special observation mode.

Preferably, the image processor generates an oxygen saturation image by calculating an oxygen saturation level on the basis of the first image capture signal and the second image capture signal, calculating a normal observation image on the basis of the first image capture signal, and performing image processing on the normal observation image on the basis of the oxygen saturation level.

According to the present invention, the first illumination light and the second illumination light having a spectral property different from that of the first illumination light and including different-absorption-wavelength light that has different absorption coefficients for oxygenated hemoglobin and reduced hemoglobin are alternately emitted to a test body while a turn-off period is interposed in the special observation mode. The first read time taken to read the first image capture signal in the turn-off period after emission of the first illumination light is made shorter than the second read time taken to read the second image capture signal in the turn-off period after emission of the second illumination light. Accordingly, it is possible to suppress a decrease in the frame rate without a decrease in the brightness and resolution of an oxygen saturation image generated on the basis of the first image capture signal and the second image capture signal.

### BRIEF DESCRIPTION OF THE DRAWINGS

Fig. 1 is an external view of an endoscope system;
Fig. 2 is a front view of a distal end section of an endoscope;
Fig. 3 is a block diagram illustrating an electrical configuration of the endoscope system;
Fig. 4 is a graph illustrating the intensity spectra of first and second illumination light;
Fig. 5 is a diagram illustrating an electrical configuration of an image sensor;
Figs. 6A to 6C are diagrams for describing an operation of a column ADC circuit;
Fig. 7 is a diagram illustrating a configuration of a color filter array;
Fig. 8 is a diagram illustrating the spectral transmission properties of color filters;
Fig. 9 is a diagram for describing exposure and signal read timings in a normal observation mode and in a special observation mode;
Fig. 10 is a block diagram illustrating a configuration of an oxygen saturation image generation unit;
Fig. 11 is a graph illustrating correlations between signal ratios B2/G1 and R1/G1 and oxygen saturation levels;
Fig. 12 is a graph illustrating the absorption coefficients of oxygenated hemoglobin and reduced hemoglobin;
Fig. 13 is a flowchart illustrating an operation of the endoscope system;
Fig. 14 is a diagram for describing first and second pixel areas;
Figs. 15A to 15C are diagrams for describing an operation of the column ADC circuit for reducing a signal read time;
Fig. 16 is a diagram for describing an example in which the exposure time of the second illumination light is made shorter than the exposure time of the first illumination light; and
Fig. 17 is a diagram illustrating a configuration of a capsule endoscope.

### DESCRIPTION OF THE PREFERRED EMBODIMENTS

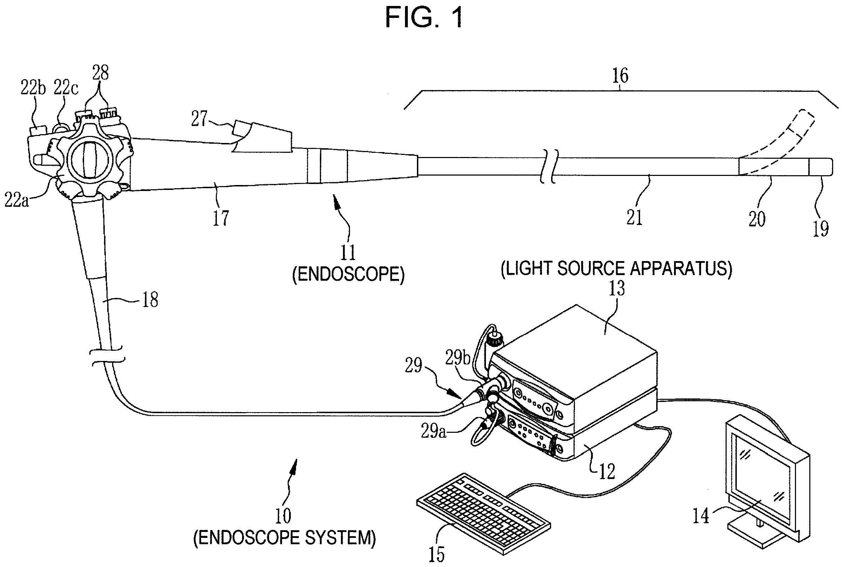

In Fig. 1, an endoscope system 10 includes an endoscope 11 that captures an image of an observation region (test body) in a living body, a processor apparatus 12 that generates a display image of the observation region on the basis of an image capture signal obtained as a result of image capture, a light source apparatus 13 that supplies illumination light for illuminating the observation region to the endoscope 11, and a monitor 14 that displays the display image. To the processor apparatus 12, the monitor 14 and an input unit 15, such as a keyboard or a mouse, are connected.

The endoscope 11 includes an insertion section 16 that is inserted into the alimentary canal of the living body, an operation section 17 that is provided to the base end portion of the insertion section 16, and a universal cord 18 that is used to connect the endoscope 11 with the processor apparatus 12 and the light source apparatus 13. The insertion section 16 is constituted by a distal end section 19, a bending section 20, and a flexible tube section 21, which are coupled to each other in this order from the distal end side.

The operation section 17 is provided with an angulation knob 22a, a mode switching switch 22b, and so on. The angulation knob 22a is used in an operation for bending the bending section 20. By operating the angulation knob 22a, the distal end section 19 can be turned in a desired direction.

The mode switching switch 22b is used in a switching operation between two types of observation modes, namely, a normal observation mode and a special observation mode. The normal observation mode is a mode in which a normal observation image obtained by capturing a full-color image of an observation target using white light is displayed on the monitor 14. The special observation mode is a mode in which the oxygen saturation level of blood hemoglobin of an observation target is calculated, and an oxygen saturation image obtained by performing image processing on a normal observation image on the basis of the oxygen saturation level is displayed on the monitor 14.

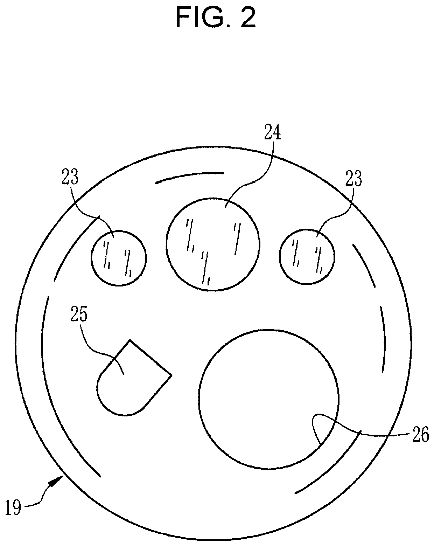

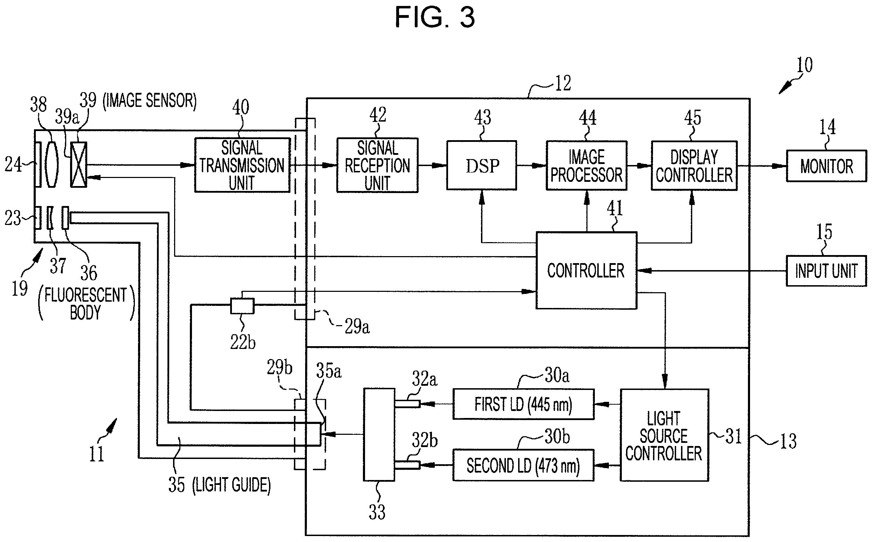

In Fig. 2, on the distal end face of the distal end section 19, illumination windows 23 through which illumination light is emitted to an observation region, an observation window 24 for taking in an image of the observation region, an air and water supply nozzle 25 for supplying air and water for cleaning the observation window 24, and a forceps outlet 26 through which medical tools, such as forceps and an electric knife, protrude for performing various treatments are provided. Behind the observation window 24, an image sensor 39 (see Fig. 3) is provided.

The bending section 20 is constituted by a plurality of bending pieces coupled to each other and bends in the up-down and right-left directions in response to an operation of the angulation knob 22a of the operation section 17. By bending the bending section 20, the distal end section 19 is turned in a desired direction. The flexible tube section 21 has flexibility and can be inserted into a winding canal, such as an esophagus and bowels. A signal cable used to transmit control signals for driving the image sensor 39 and image capture signals output from the image sensor 39, and light guides 35 (see Fig. 3) used to guide illumination light supplied from the light source apparatus 13 to the illumination windows 23 are inserted into and pass through the insertion section 16.

The operation section 17 is provided with a forceps inlet 27 through which medical tools are inserted, air and water supply buttons 28 that are operated in order to supply air and water through the air and water supply nozzle 25, a freeze button (not illustrated) used to capture a still image, and so on in addition to the angulation knob 22a and the mode switching switch 22b.

A communication cable extended from the insertion section 16 and the light guides 35 are inserted into and pass through the universal cord 18, and a connector 29 is attached to one end of the universal cord 18 on the side close to the processor apparatus 12 and the light source apparatus 13. The connector 29 is a combination-type connector constituted by a communication connector 29a and a light source connector 29b. The communication connector 29a and the light source connector 29b are detachably connected to the processor apparatus 12 and to the light source apparatus 13 respectively. At the communication connector 29a, one end of the communication cable is located. At the light source connector 29b, entrance ends 35a (see Fig. 3) of the light guides 35 are located.

In Fig. 3, the light source apparatus 13 includes a first laser diode (LD) 30a, a second LD 30b, a light source controller 31, a first optical fiber 32a, a second optical fiber 32b, and an optical coupler 33. The first LD 30a emits first blue laser light having a center wavelength of 445 nm. The second LD 30b emits second blue laser light having a center wavelength of 473 nm. The half width of the first and second blue laser light is about ±10 nm. As the first LD 30a and the second LD 30b, broad-area InGaN laser diodes, InGaNAs laser diodes, GaNAs laser diodes, and so on are used.

The light source controller 31 controls the first LD 30a and the second LD 30b to turn on and off the LDs individually. In the normal observation mode, the light source controller 31 turns on the first LD 30a. In the special observation mode, the light source controller 31 turns on the first LD 30a and the second LD 30b alternately.

The first blue laser light emitted from the first LD 30a enters the first optical fiber 32a. The second blue laser light emitted from the second LD 30b enters the second optical fiber 32b. The first optical fiber 32a and the second optical fiber 32b are connected to the optical coupler 33. The optical coupler 33 unites the optical path of the first optical fiber 32a and that of the second optical fiber 32b and causes the first blue laser light and the second blue laser light to respectively enter the entrance ends 35a of the light guides 35 of the endoscope 11.

The endoscope 11 includes the light guides 35, a fluorescent body 36, an illumination optical system 37, an image capture optical system 38, the image sensor 39, and a signal transmission unit 40. One light guide 35 is provided for each of the illumination windows 23. As the light guides 35, multimode fibers can be used. For example, small-diameter fiber cables having a core diameter of 105 µm, a clad diameter of 125 µm, and a diameter including a protective layer that serves as an outer sheath of 0.3 to 0.5 mm can be used.

When the light source connector 29b is coupled to the light source apparatus 13, the entrance ends 35a of the respective light guides 35 located at the light source connector 29b face the emission end of the optical coupler 33. The fluorescent body 36 is located so as to face the emission ends of the respective light guides 35 positioned in the distal end section 19. The first blue laser light or the second blue laser light enters the fluorescent body 36 through a corresponding one of the light guides 35.

The fluorescent body 36 is formed by dispersing a plurality of types of fluorescent materials (YAG fluorescent materials or BAM (BaMgAl₁₀O₁₇) fluorescent materials, for example) in a binder and forming the fluorescent materials in a rectangular parallelepiped form. The fluorescent body 36 absorbs a portion of laser light (the first blue laser light or the second blue laser light) entering through a corresponding one of the light guides 35, is excited, and emits fluorescence having a wavelength band ranging from green to red. The portion of the laser light that enters the fluorescent body 36 passes through the fluorescent body 36 without being absorbed by the fluorescent body 36. As a result, the fluorescence and the portion of the laser light are emitted from the fluorescent body 36.

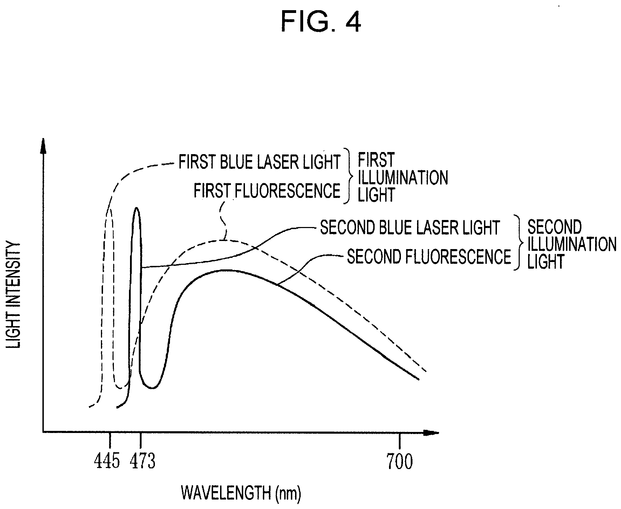

Specifically, in a case where the first LD 30a is turned on and the first blue laser light enters the fluorescent body 36, first illumination light having a spectrum illustrated in Fig. 4 is emitted from the fluorescent body 36. The first illumination light includes the first blue laser light and first fluorescence emitted from the fluorescent body 36 as a result of excitation by the first blue laser light. In a case where the second LD 30b is turned on and the second blue laser light enters the fluorescent body 36, second illumination light having a spectrum illustrated in Fig. 4 is emitted from the fluorescent body 36. The second illumination light includes the second blue laser light and second fluorescence emitted from the fluorescent body 36 as a result of excitation by the second blue laser light and has a spectral property different from that of the first illumination light. The spectral shapes of the first fluorescence and the second fluorescence are substantially the same. That is, the ratio between the intensity I₁(λ) of the first fluorescence and the intensity I₂(λ) of the second fluorescence at a wavelength λ is substantially constant.

The first illumination light and the second illumination light emitted from the fluorescent body 36 are condensed by the illumination optical system 37 and are emitted to an observation region in a living body via the illumination windows 23. Reflection light from the observation region enters the image capture optical system 38 via the observation window 24, and an image is formed on an image capture face 39a of the image sensor 39 by the image capture optical system 38. In this embodiment, the light source apparatus 13, the light guides 35, the fluorescent body 36, and the illumination optical system 37 correspond to an illumination unit described in the appended claims.

The image sensor 39 is of CMOS type that captures an image of the reflection light from the observation region and outputs an image capture signal on the basis of an image capture control signal supplied from the processor apparatus 12. Hereinafter, an image capture signal obtained by the image sensor 39 capturing an image of reflection light from an observation region illuminated with the first illumination light is referred to as a first image capture signal, and an image capture signal obtained by the image sensor 39 capturing an image of reflection light from the observation region illuminated with the second illumination light is referred to as a second image capture signal.

The signal transmission unit 40 transmits the first and second image capture signals obtained by the image sensor 39 to the processor apparatus 12 in accordance with a known low-voltage-operating signaling transmission method. When the above-described mode switching switch 22b provided to the endoscope 11 is operated, a mode switching operation signal is transmitted to the processor apparatus 12 from the mode switching switch 22b.

The processor apparatus 12 includes a controller 41, a signal reception unit 42, a digital signal processor (DSP) 43, an image processor 44, and a display controller 45. The controller 41 controls the components in the processor apparatus 12 and also controls the image sensor 39 of the endoscope 11 and the light source controller 31 of the light source apparatus 13.

The signal reception unit 42 receives the first and second image capture signals transmitted from the signal transmission unit 40 of the endoscope 11. The DSP 43 performs known signal processing on the first and second image capture signals received by the signal reception unit 42, such as a defect correction process, a gain correction process, a white balance process, gamma conversion, and a synchronization process.

The image processor 44 generates a normal observation image in the normal observation mode by performing a color conversion process, a color enhancement process, a structure enhancement process, and the like on the first image capture signal on which signal processing has been performed by the DSP 43. The image processor 44 generates an oxygen saturation image in the special observation mode by calculating the oxygen saturation level on the basis of the first and second image capture signals on which signal processing has been performed by the DSP 43, calculating a normal observation image on the basis of the first image capture signal, and performing image processing on the normal observation image on the basis of the oxygen saturation level.

The display controller 45 converts the image generated by the image processor 44 into a signal in a display form and causes the monitor 14 to display the image.

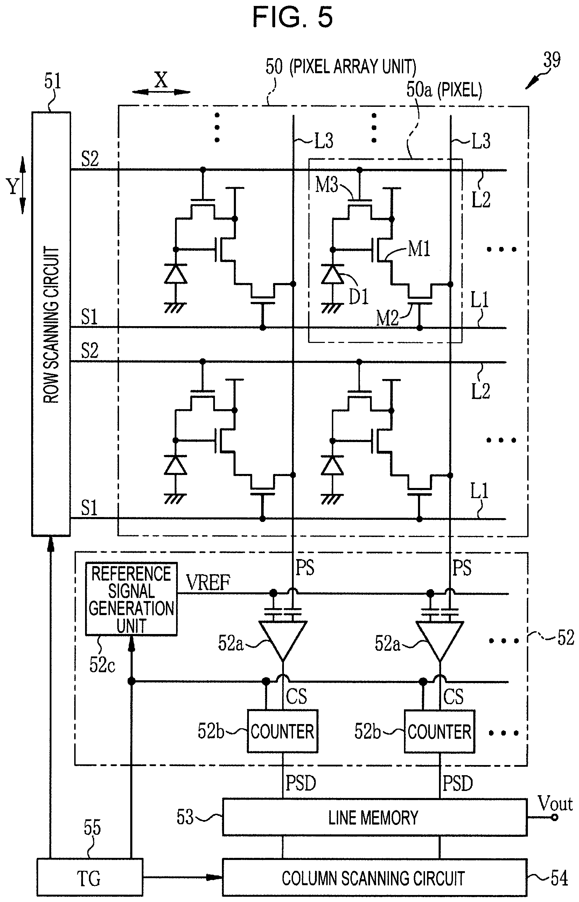

In Fig. 5, the image sensor 39 includes a pixel array unit 50, a row scanning circuit 51, a column ADC circuit 52 including a plurality of ADCs (analog-to-digital converters) that are arranged in a row direction, a line memory 53, a column scanning circuit 54, and a timing generator (TG) 55. In the image sensor 39, an ADC is arranged for each column of pixels. The pixel array unit 50 is constituted by a plurality of pixels 50a that are arranged in a two dimensional matrix in the row direction (X direction) and in a column direction (Y direction), and is provided on the image capture face 39a described above. In the pixel array unit 50, row selection lines L1 and row reset lines L2 are laid in the row direction, and column signal lines L3 are laid in the column direction. Each of the pixels 50a is connected to a corresponding one of the row selection lines L1, a corresponding one of the row reset lines L2, and a corresponding one of the column signal lines L3. The TG 55 controls each component on the basis of control signals input from the controller 41 of the processor apparatus 12.

Each of the pixels 50a includes a photodiode D1, an amplifier transistor M1, a pixel selection transistor M2, and a reset transistor M3. The photodiode D1 performs photoelectric conversion on incident light, generates a signal charge that corresponds to the amount of incident light, and accumulates the signal charge. The amplifier transistor M1 converts the signal charge accumulated by the photodiode D1 into a voltage value (pixel signal PS). The pixel selection transistor M2 is controlled by a corresponding one of the row selection lines L1 and causes the pixel signal PS generated by the amplifier transistor M1 to be output to a corresponding one of the column signal lines L3. The reset transistor M3 is controlled by a corresponding one of the row reset lines L2 and releases into a power supply line (resets) the signal charge accumulated by the photodiode D1.

The row scanning circuit 51 generates a row selection signal S1 and a reset signal S2 on the basis of timing signals input from the TG 55. The row scanning circuit 51 applies the row selection signal S1 to any of the row selection lines L1 upon a signal read operation to thereby cause the pixel signals PS of the pixels 50a connected to the row selection line L1 to be output to the column signal lines L3. The row scanning circuit 51 applies the reset signal S2 to any of the row reset lines L2 upon a reset operation to thereby reset the pixels 50a connected to the row reset line L2.

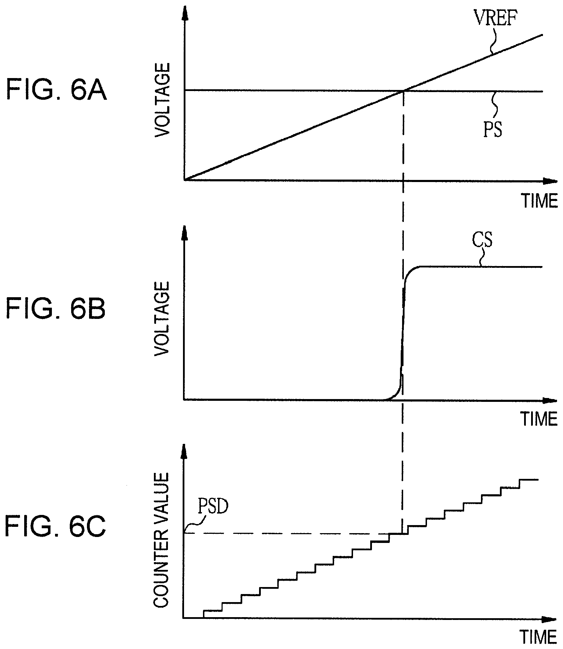

The column ADC circuit 52 includes comparators 52a, counters 52b, and a reference signal generation unit 52c. The comparators 52a and the counters 52b are connected to the column signal lines L3. The reference signal generation unit 52c generates a reference signal VREF having a voltage that linearly increases as time passes, as illustrated in Fig. 6A, on the basis of a clock signal input from the TG 55. To each of the comparators 52a, the pixel signal PS is input via a corresponding one of the column selection lines L3, and the reference signal VREF is input from the reference signal generation unit 52c.

Each of the comparators 52a compares the pixel signal PS with the reference signal VREF and outputs a signal CS that indicates the magnitude relationship between the voltage values of the signals, as illustrated in Fig. 6B. The output signal CS is input to a corresponding one of the counters 52b. The counter 52b starts a count operation when the voltage of the reference signal VREF starts increasing, as illustrated in Fig. 6C, on the basis of the clock signal input from the TG 55. When the voltage value of the pixel signal PS matches the voltage value of the reference signal VREF, and the output signal CS changes from a low level to a high level, the counter 52b stops the count operation. A count value at the time when the counter 52b stops the count operation corresponds to the pixel signal PS. The count value is a digital signal and is output to the line memory 53 from the column ADC circuit 52 as a digitized pixel signal PSD.

The line memory 53 collectively retains the pixel signals PSD for one row digitized by the column ADC circuit 52. The column scanning circuit 54 scans the line memory 53 on the basis of a timing signal input from the TG 55 to thereby cause the pixel signals PSD to be sequentially output from an output terminal Vout. The pixel signals PSD for one frame output from the output terminal Vout correspond to the first image capture signal or the second image capture signal described above.

The TG 55 generates timing signals on the basis of image capture control signals input from the controller 41 of the processor apparatus 12. As a method for reading the image capture signals, a "sequential read method" and a "skip read method" can be performed. In the sequential read method, the row scanning circuit 51 sequentially selects each of the row selection lines L1 and applies the row selection signal S1 to thereby perform signal reading from the pixel rows one by one sequentially. In contrast, in the skip read method, the row scanning circuit 51 sequentially selects the row selection line L1 every other pixel row and applies the row selection signal S1, and the column scanning circuit 54 scans the line memory 53 every other pixel column to thereby perform signal reading while skipping every other pixel row in the column direction and skipping every other pixel column in the row direction. Here, the pixel row corresponds to the pixels 50a for one row that are arranged in the row direction, and the pixel column corresponds to the pixels 50a for one column that are arranged in the column direction.

As a method for resetting the image capture signals, a "sequential reset method" and a "collective reset method" can be performed. In the sequential reset method, the row scanning circuit 51 sequentially selects each of the row reset lines L2 and applies the reset signal S2 to thereby reset the pixel rows one by one sequentially. In contrast, in the collective reset method, the row scanning circuit 51 selects all of the row reset lines L2 and applies the reset signal S2 to all of the row reset lines L2 collectively to thereby reset all of the pixel rows simultaneously.

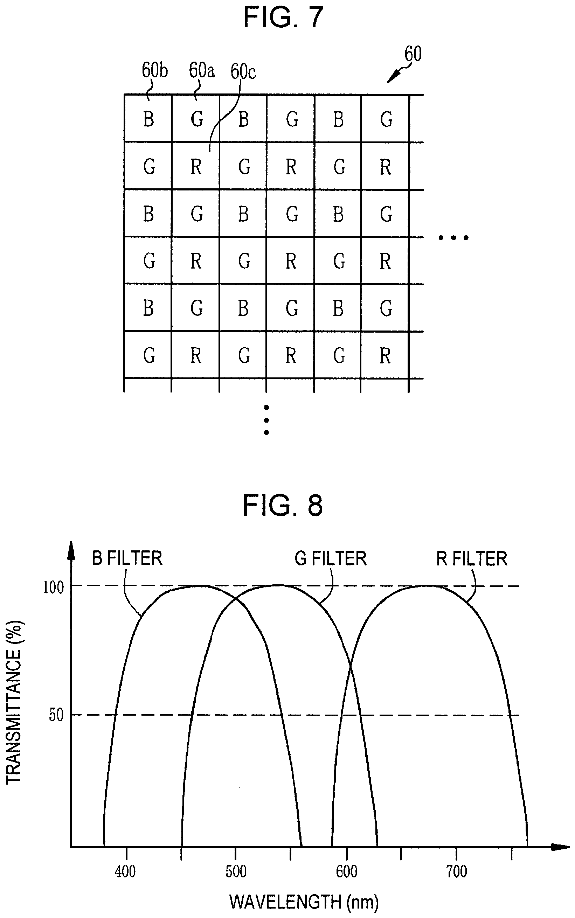

As illustrated in Fig. 7, a color filter array 60 is provided on the light entrance side of the pixel array unit 50. The color filter array 60 includes green (G) filters 60a, blue (B) filters 60b, and red (R) filters 60c. On each of the pixels 50a, a corresponding one of these filters is arranged. The color arrangement of the color filter array 60 is based on the Bayer arrangement, in which the G filters 60a are arranged every other pixel in a checkered pattern, and the B filters 60b and the R filters 60c are arranged on the remaining pixels so that the B filters 60b and the R filters 60c are in square grid patterns respectively.

As illustrated in Fig. 8, the G filters 60a have a high transmittance in a wavelength range between 450 and 630 nm or so. The B filters 60b have a high transmittance in a wavelength range between 380 and 560 nm or so. The R filters 60c have a high transmittance in a wavelength range between 580 and 760 nm or so. Hereinafter, the pixels 50a on which the G filters 60a are arranged are referred to as G pixels, the pixels 50a on which the B filters 60b are arranged are referred to as B pixels, and the pixels 50a on which the R filters 60c are arranged are referred to as R pixels. These G, B, and R pixels correspond to a "plurality of types of pixel groups having different spectral sensitivities" described in the appended claims. Among these pixels, a group of pixels of highest sensitivity to different-absorption-wavelength light described below corresponds to the B pixels.

When the first illumination light is emitted, the first blue laser light and a shorter-wavelength component of the first fluorescence enter the B pixels, a main-wavelength component of the first fluorescence enters the G pixels, and a longer-wavelength component of the first fluorescence enters the R pixels. Similarly, when the second illumination light is emitted, the second blue laser light and a shorter-wavelength component of the second fluorescence enter the B pixels, a main-wavelength component of the second fluorescence enters the G pixels, and a longer-wavelength component of the second fluorescence enters the R pixels. Note that the emission intensity of the first blue laser light is larger than that of the first fluorescence, and the emission intensity of the second blue laser light is larger than that of the second fluorescence. Therefore, most of the light that enters the B pixels is formed of a component of the first blue laser light or a component of the second blue laser light.

As described above, the image sensor 39 is a single-panel-type color image sensor, and therefore, each of the first and second image capture signals described above is divided into G, B, and R pixel signals. Hereinafter, the G, B, and R pixel signals included in the first image capture signal are respectively referred to as G1 pixel signals, B1 pixel signals, and R1 pixel signals. The G, B, and R pixel signals included in the second image capture signal are respectively referred to as G2 pixel signals, B2 pixel signals, and R2 pixel signals.

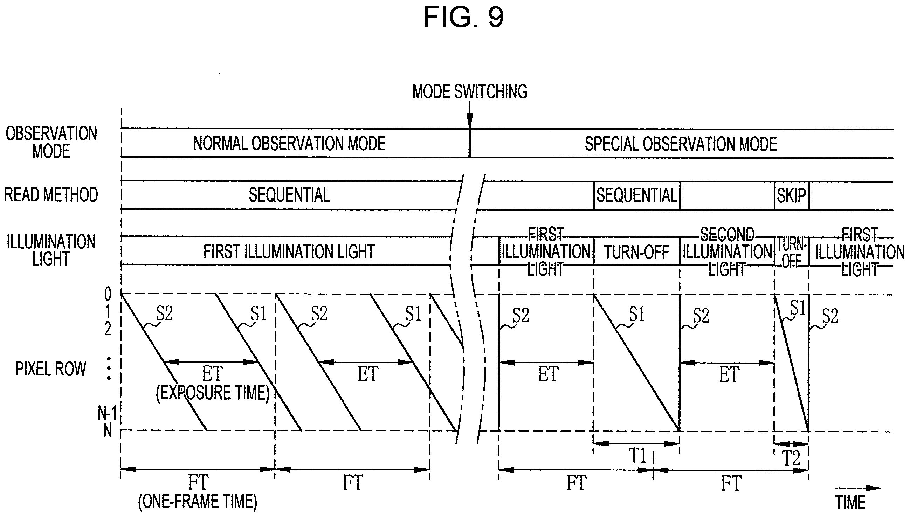

Now, control performed by the controller 41 in accordance with the observation mode is described. As illustrated in Fig. 9, in the normal observation mode, the controller 41 controls the light source controller 31 to turn on the first LD 30a, thereby causing the first illumination light to be emitted through a corresponding one of the illumination windows 23 of the endoscope 11. In a state where the first illumination light is being emitted, the controller 41 controls and drives the image sensor 39 in accordance with the rolling shutter method.

Specifically, resetting is first performed for the pixel rows from the first pixel row "0" to the last pixel row "N" one by one sequentially in accordance with the sequential reset method. After an exposure time ET has passed since the start of the sequential resetting, signal reading is performed for the pixel rows from the first pixel row "0" to the last pixel row "N" one by one sequentially in accordance with the sequential read method. As a result, a first image capture signal for one frame is output from the image sensor 39. This driving in accordance with the rolling shutter method is repeatedly performed during the normal observation mode, and a first image capture signal for one frame is obtained for each one-frame time FT.

When the mode switching switch 22b is operated during the normal observation mode and the controller 41 receives a mode switching operation signal for giving an instruction for switching from the normal observation mode to the special observation mode, the controller 41 controls the light source controller 31 to turn on the first LD 30a and the second LD 30b alternately while interposing a turn-off period, thereby causing the first illumination light and the second illumination light to be emitted through the respective illumination windows 23 of the endoscope 11 alternately while interposing the turn-off period. The controller 41 controls and drives the image sensor 39 in accordance with a global shutter method while performing switching between the first and second illumination light.

Specifically, in a state where the first illumination light is being emitted through a corresponding one of the illumination windows 23 of the endoscope 11, all of the pixel rows are simultaneously reset first in accordance with the collective reset method. After the exposure time ET has passed since the performance of the collective resetting, emission of the first illumination light is stopped, thereby causing both the first LD 30a and the second LD 30b to enter a turn-off state. During this turn-off period, signal reading is performed for the pixel rows from the first pixel row "0" to the last pixel row "N" one by one sequentially in accordance with the sequential read method. As a result, a first image capture signal for one frame is obtained.

Next, the second LD 30b is turned on to emit the second illumination light, and all of the pixel rows are simultaneously reset in accordance with the collective reset method. After the exposure time ET has passed since the performance of the collective resetting, emission of the second illumination light is stopped, thereby causing both the first LD 30a and the second LD 30b to enter the turn-off state. During this turn-off period, signal reading is performed every other line in the row direction and in the column direction in accordance with the skip read method. In this skip reading, only B2 pixel signals present on even-numbered rows and even-numbered columns are read. As a result, only the B2 pixel signals are quickly read as a second image capture signal for one frame.

As described above, in the special observation mode, skip reading is performed to thereby make a second read time T2 taken to read the second image capture signal shorter than a first read time T1 taken to read the first image capture signal. The first read time T1 is equal to the time taken to read the first image capture signal in the normal observation mode.

The global shutter method is used in the special observation mode, and therefore, the sum of the exposure time ET of the first illumination light and the first read time T1 is longer than the frame time FT (1/60 seconds, for example) in the normal observation mode. However, the second read time T2 is made shorter than the first read time T1, and therefore, the sum of the exposure time ET of the second illumination light and the second read time T2 is shorter than the frame time FT. Accordingly, the time taken to obtain the first and second image capture signals can be made equal to twice the frame time FT in the special observation mode.

When the image sensor 39 is driven as described above, the first image capture signal that includes the B1, G1, and R1 pixel signals and the second image capture signal that only includes the B2 pixel signals are input to the DSP 43. The DSP 43 performs a color interpolation process as the synchronization process and generates one set of B1, G1, R1, and B2 pixel signals per pixel.

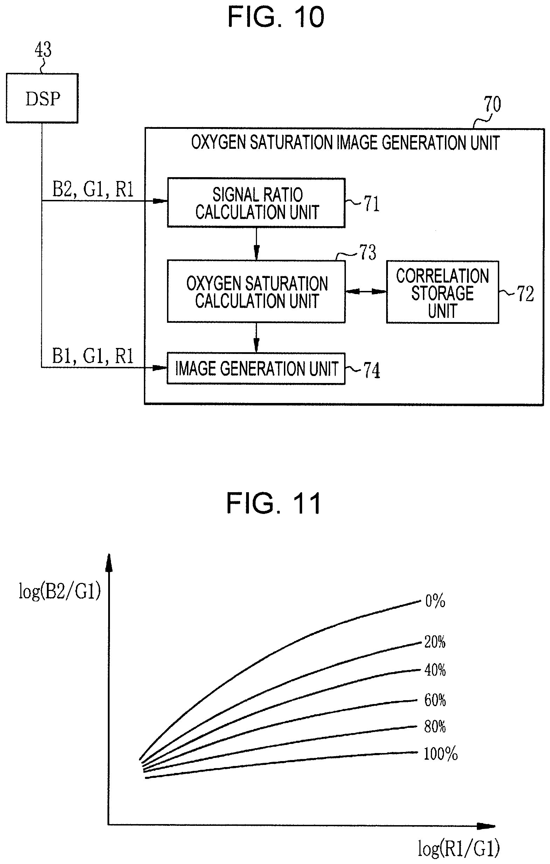

The image processor 44 of the processor apparatus 12 includes an oxygen saturation image generation unit 70 illustrated in Fig. 10. The oxygen saturation image generation unit 70 includes a signal ratio calculation unit 71, a correlation storage unit 72, an oxygen saturation calculation unit 73, and an image generation unit 74.

To the signal ratio calculation unit 71, the B2 pixel signals, the G1 pixel signals, and the R1 pixel signals in the first and second image capture signals input from the DSP 43 to the image processor 44 are input. The signal ratio calculation unit 71 calculates for each pixel a signal ratio B2/G1 between the B2 pixel signal and the G1 pixel signal and a signal ratio R1/G1 between the R1 pixel signal and the G1 pixel signal. These signal ratios B2/G1 and R1/G1 are used to calculate the oxygen saturation level. A signal ratio essential for calculating the oxygen saturation level is B2/G1.

The correlation storage unit 72 stores correlations between the signal ratios B2/G1 and R1/G1 and oxygen saturation levels. These correlations are stored as a 2D table in which the isopleths of the oxygen saturation levels are defined in 2D space, as illustrated in Fig. 11. The positions and shapes of the isopleths relative to the signal ratios B2/G1 and R1/G1 are obtained in advance by performing a physical simulation of light scattering, and the interval between the isopleths changes in accordance with the blood volume (the signal ratio R1/G1). Note that the correlations between the signal ratios B2/G1 and R1/G1 and the oxygen saturation levels are stored on a logarithmic scale.

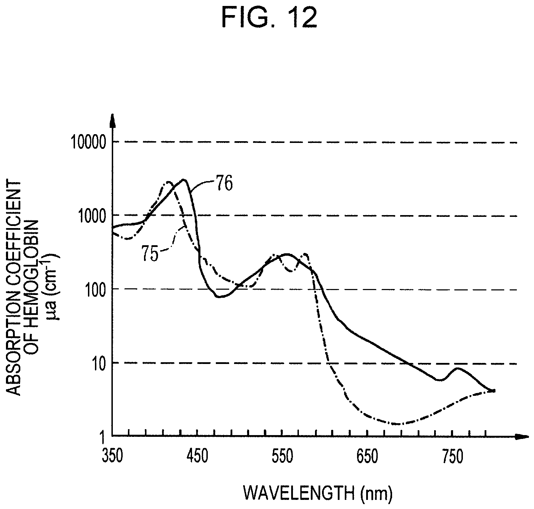

The above-described correlations closely relate to the absorption property of oxygenated hemoglobin (represented by the dotted-chain line 75) and the absorption property of reduced hemoglobin (represented by the solid line 76) illustrated in Fig. 12. An oxygen saturation level can be calculated by using light (different-absorption-wavelength light) having a certain wavelength, such as the second blue laser light having a center wavelength of 473 nm, which causes a large difference between the absorption coefficient of oxygenated hemoglobin and the absorption coefficient of reduced hemoglobin. However, the B2 pixel signals that mainly depend on the second blue laser light largely depend not only on the oxygen saturation level but also on the blood volume. In contrast, the R1 pixel signals mainly depend on the blood volume. Therefore, by using the values (the signal ratios B2/G1 and R1/G1) obtained by dividing each of the B2 pixel signals and each of the R1 pixel signals by each of the G1 pixel signals, which serves as a reference signal, the oxygen saturation level can be obtained with high accuracy while the dependence on the blood volume is reduced.

The oxygen saturation calculation unit 73 refers to the correlations stored on the correlation storage unit 72 and calculates the oxygen saturation level that corresponds to the signal ratios B2/G1 and R1/G1 calculated by the signal ratio calculation unit 71 for each pixel. The calculated value of the oxygen saturation level scarcely falls below 0% or exceeds 100%. In a case where the calculated value falls below 0%, the oxygen saturation level can be assumed to be 0%. In a case where the calculated value exceeds 100%, the oxygen saturation level can be assumed to be 100%.

The image generation unit 74 performs image processing on a normal observation image generated from the first image capture signal (the B1, G1, and R1 pixel signals) by using the oxygen saturation levels calculated by the oxygen saturation calculation unit 73. Specifically, the image generation unit 74 performs gain correction on each of the B1, G1, and R1 pixel signals in accordance with the oxygen saturation level. For example, for a pixel for which the correction oxygen saturation level is 60% or more, the gain is set to "1" for all of the B1, G1, and R1 pixel signals. For a pixel for which the correction oxygen saturation level is less than 60%, the gain is set to a value smaller than "1" for the B1 pixel signal, and the gain is set to a value equal to or larger than "1" for the G1 and R1 pixel signals. Then, the B1, G1, and R1 pixel signals obtained after gain correction are used to generate an image. This image is an oxygen saturation image. In this oxygen saturation image, a high oxygen area (an area having an oxygen saturation level between 60 and 100%) is colored similarly to the normal observation image, and the color of a low oxygen area (an area having an oxygen saturation level between 0 and 60%) is changed to blue.

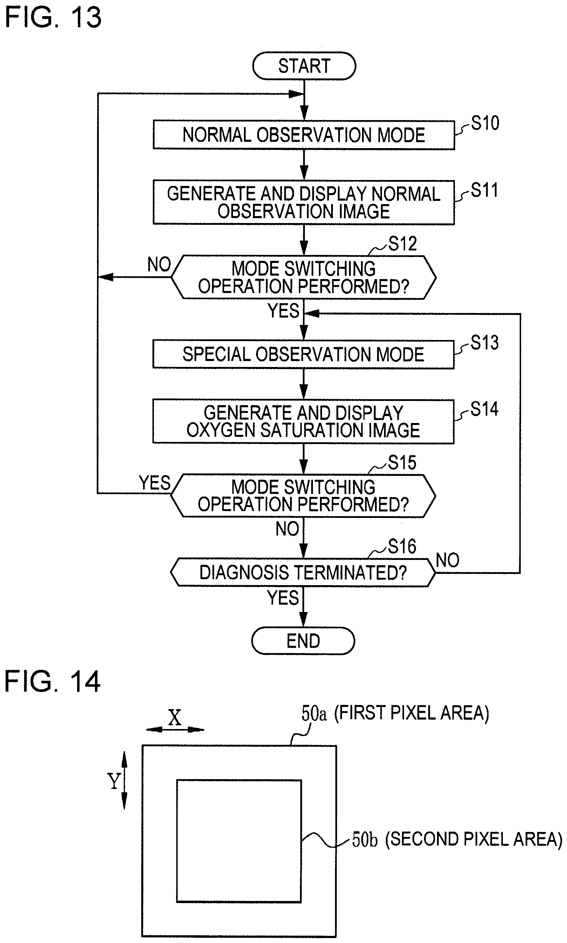

Now, an operation of the endoscope system 10 is described with reference to the flowchart in Fig. 13. First, an operator inserts the endoscope 11 into a living body and observes an observation region in the normal observation mode (step S10). In the normal observation mode, the image sensor 39 is driven in accordance with the rolling shutter method in a state where the first illumination light is being emitted to the observation region, and a first image capture signal is read, as illustrated in Fig. 9. A first image capture signal is read for each one-frame time. On the basis of the first image capture signal read by the image sensor 39, a normal observation image is generated by the image processor 44 and is displayed on the monitor 14 (step S11). The display frame rate of the monitor 14 is equal to the frame rate of the image sensor 39, and the normal observation image displayed on the monitor 14 is refreshed for each one-frame time.

When the operator finds a region that is likely to be a lesion as a result of observation in the normal observation mode and operates the mode switching switch 22b to switch the observation mode (Yes in step S12), the observation mode transitions to the special observation mode (step S13). In the special observation mode, the first illumination light and the second illumination light are alternately emitted to the observation region while a turn-off period is interposed, and the image sensor 39 is driven in accordance with the global shutter method, as illustrated in Fig. 9.

Here, reading of a first image capture signal after emission of the first illumination light is performed in accordance with the sequential read method, and reading of a second image capture signal after emission of the second illumination light is performed in accordance with the skip read method. Reading of first and second image capture signals is performed for each unit time that corresponds to a two-frame time. On the basis of the first and second image capture signals, an oxygen saturation image is generated by the oxygen saturation image generation unit 70 of the image processor 44 and is displayed on the monitor 14 (step S14). The display frame rate of the monitor 14 is equal to the frame rate of the image sensor 39, and the oxygen saturation image displayed on the monitor 14 is refreshed for each two-frame time.

Generation and display of an oxygen saturation image are repeatedly performed until the operator operates the mode switching switch 22b again or performs an operation for terminating the diagnosis. If the mode switching switch 22b is operated (Yes in step S15), the observation mode returns to the normal observation mode (step S10), and a similar operation is performed. On the other hand, if the mode switching switch 22b is not operated and an operation for terminating the diagnosis is performed (Yes in step S16), the operation of the endoscope system 10 is terminated.

As described above, the endoscope system 10 uses the image sensor 39 of CMOS type. Therefore, in the special observation mode in which alternate switching between the first and second illumination light is performed, a turn-off period is provided upon switching between the first and second illumination light in order to prevent the first illumination and the second illumination light from mixing during the exposure period for each pixel row, and reading of first and second image capture signals is performed during the turn-off periods. Accordingly, providing the turn-off period simply results in a decrease in the frame rate. In this embodiment, however, the second read time T2 of the second image capture signal is reduced by performing skip reading, and therefore, a decrease in the frame rate is suppressed. Only the B2 pixel signals in the second image capture signal are necessary to generate an oxygen saturation image, and therefore, the brightness and resolution do not decrease even if skip reading is performed.

Note that, in the above-described embodiment, the second read time T2 taken to read the second image capture signal is reduced by performing skip reading; however, when the second image capture signal is read, the clock frequency of the control signal input to the TG 55 from the controller 41 may be increased to thereby reduce the second read time T2.

Further, when the second image capture signal is read, addition for each set of two pixels (addition of pixel signals of the same color) may be performed in the column direction instead of skip reading to thereby reduce the second read time T2. A method for pixel addition in a CMOS image sensor is made public by Japanese Unexamined Patent Application Publication No. 2012-253624, for example. The pixel addition includes average addition.

In the above-described embodiment, pixel signals are read from the entire area of the pixel array unit 50; however, when the second image capture signal is read, pixel signals may be read only from a second pixel area (reduced area) 50b obtained by reducing the first pixel area (whole area) 50a that corresponds to the pixel array unit 50 in the row direction and in the column direction, as illustrated in Fig. 14, to thereby reduce the second read time T2. The second pixel area 50b may be an area obtained as a result of reduction only in one of the row direction and the column direction.

Further, when the second image capture signal is read, the temporal change rate of the reference signal VREF used by the column ADC circuit 52 can be made larger than that when the first image capture signal is read to thereby reduce the second read time T2.

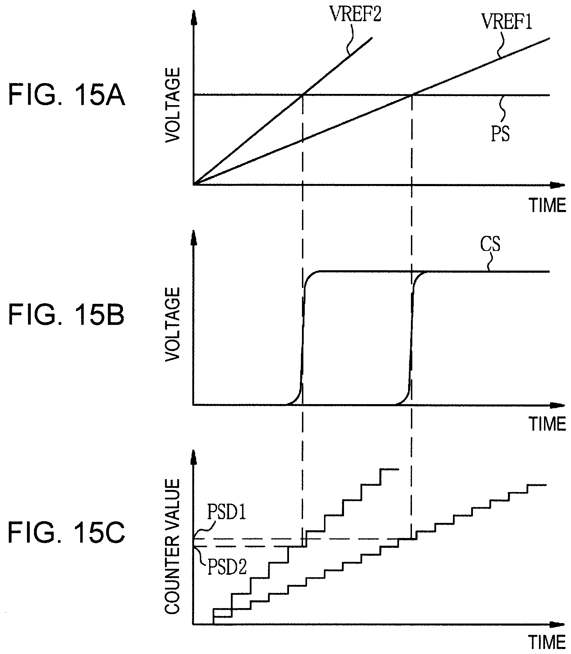

Specifically, output of a first reference signal VREF1 and a second reference signal VREF2 from the reference signal generation unit 52c is enabled, as illustrated in Fig. 15A. The first reference signal VREF1 is used when the first image capture signal is read, and the second reference signal VREF2 is used when the second image capture signal is read. The temporal change rate of the second reference signal VREF2 is set to a value larger than the temporal change rate of the first reference signal VREF1. The number counted by the counter 52b per clock changes in accordance with the temporal change rate of the second reference signal VREF2, as illustrated in Fig. 15C. The temporal change rate of the second reference signal VREF2 is set twice as high as the temporal change rate of the first reference signal VREF1, for example. The number counted per clock when the second reference signal VREF2 is used is set twice as high as that when the first reference signal VREF1 is used. With such a configuration, the second read time T2 becomes shorter than the first read time T1. However, a digital pixel signal PSD2 obtained by using the second reference signal VREF2 has lower accuracy than a digital pixel signal PSD1 obtained by using the first reference signal VREF1 (the accuracy of A/D conversion decreases).

When the second image capture signal is read, the blanking intervals (the vertical blanking interval and the horizontal blanking interval) can be reduced to thereby reduce the second read time T2.

Two or more methods among the above-described methods for reducing the second read time T2 of the second image capture signal may be combined to reduce the read time T2 to a maximum extent.

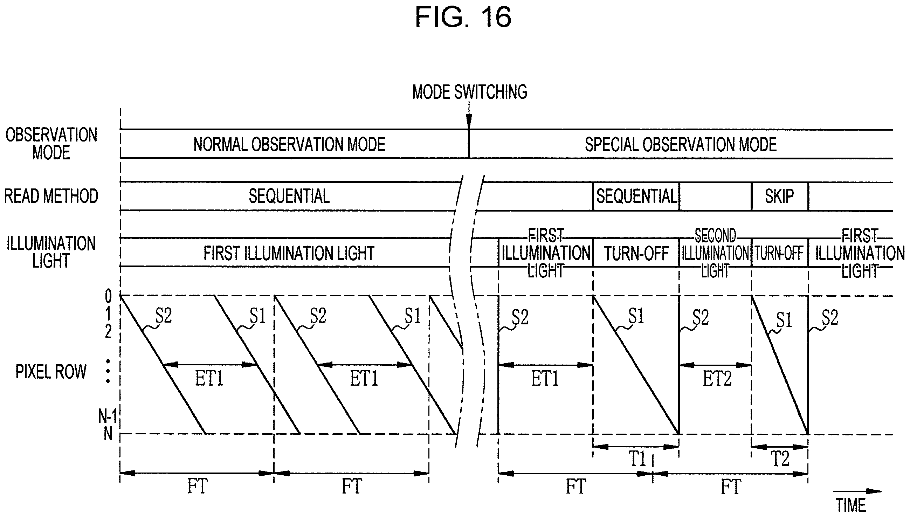

In a case where the time taken to obtain the first and second image capture signals exceeds a value twice the frame time FT even if the second read time T2 of the second image capture signal is reduced, the emission time (exposure time) ET2 of the second illumination light may be made shorter than the emission time (exposure time) ET1 of the first illumination light, as illustrated in Fig. 16 to thereby make the time taken to obtain the first and second image capture signals equal to twice the frame time FT. In this case, the brightness of the second image capture signal decreases, and therefore, it is desirable to perform gain correction on the second image capture signal. The gain correction is performed by the DSP 43. Alternatively, gain correction may be performed by a column amplifier, which is provided to the image sensor 39.

In the above-described embodiment, in the special observation mode, all pixel rows are simultaneously reset in accordance with the collective reset method upon the start of emission of the first illumination light and upon the start of emission of the second illumination light; however, it is not essential to perform collective resetting simultaneously with the start of emission of the first illumination light and with the start of emission of the second illumination light, and collective resetting may be shifted before or after the start of emission. That is, the collective resetting may be performed in association with the start of emission.

Alternatively, collective resetting need not be performed and resetting may be performed during the turn-off periods in accordance with the sequential reset method. In the turn-off period during which sequential reading for the first image capture signal is performed, each pixel row may be reset sequentially immediately after reading has been performed therefrom. In the turn-off period during which skip reading for the second image capture signal is performed, each set of two pixel rows including a pixel row from which reading has just been performed and a pixel row that has been skipped due to skip reading may be reset sequentially. In this case, exposure is started in response to the start of emission of the first illumination light and to the start of emission of the second illumination light.

In the above-described embodiment, the color filter array 60 of primary color type is used; however, this type of color filter array may be replaced and a complementary-color-type color filter array may be used.

In the above-described embodiment, the first laser light emitted from the first LD 30a and the second laser light emitted from the second LD 30b are emitted to the fluorescent body 36 to thereby generate the first and second illumination light; however, the first and second illumination light may be generated by using a white light source, such as a xenon lamp, and a wavelength separation filter as disclosed by Japanese Unexamined Patent Application Publication No. 2013-165776. Further, it is possible to generate the first and second illumination light by using LEDs (three types of LEDs that respectively emit R, G, and B light, for example) and a wavelength selection filter.

In the above-described embodiment, the light source apparatus and the processor apparatus are configured separately; however, the light source apparatus and the processor apparatus may be configured as a single apparatus.

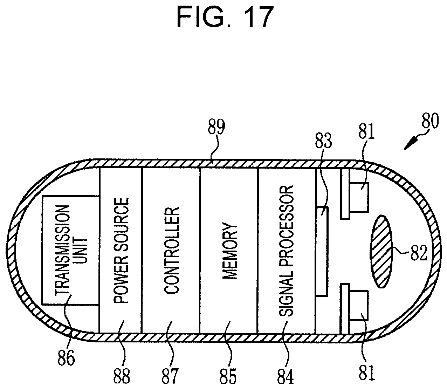

The present invention is applicable to a capsule endoscope that captures images while passing through an alimentary canal and transfers the captured images to a recording apparatus. For example, a capsule endoscope 80 is constituted by an illumination unit 81, a lens 82, an image sensor 83, a signal processor 84, a memory 85, a transmission unit 86, a controller 87, a power source 88, and a capsule housing 89 that houses these components, as illustrated in Fig. 17.

The illumination unit 81 includes an LED and a wavelength selection filter and emits the above-described first and second illumination light to a test body. The image sensor 83 is a CMOS image sensor that captures, via the lens 82, images of reflection light from the test body illuminated with the first and second illumination light and outputs the above-described first and second image capture signals. The signal processor 84 performs signal processing, which is performed by the DSP 43 and the image processor 44 in the above-described embodiment, on the first and second image capture signals and generates a normal observation image and an oxygen saturation image. The memory 85 stores the images. The transmission unit 86 wirelessly transmits the images stored in the memory 85 to an external recording apparatus (not illustrated). The controller 87 controls the components.

Note that the first and second image capture signals may be transmitted from the transmission unit 86 to an external apparatus (not illustrated), and the external apparatus may generate a normal observation image and an oxygen saturation image.

The present invention is applicable to a fiberscope in which reflection light from an observation region resulting from illumination light is guided by an image guide and to an endoscope system using an ultrasonic endoscope having a distal end into which an image sensor and an ultrasonic transducer are built.

### Reference Signs List

- 10: endoscope system
- 11: endoscope
- 12: processor apparatus
- 13: light source apparatus
- 30a: first laser diode
- 30b: second laser diode
- 35: light guide
- 36: fluorescent body
- 39: image sensor
- 41: controller
- 50: pixel array unit
- 50a: pixel
- 52: column ADC circuit

## Claims

1. An endoscope system (10) comprising:
an illumination unit (13) that is configured to irradiate a test body with first illumination light and second illumination light having a spectral property different from a spectral property of the first illumination light and including different-absorption-wavelength light that has different absorption coefficients for oxygenated hemoglobin and reduced hemoglobin;
an endoscope (11) including a CMOS image sensor (39) that is configured to capture an image of the test body illuminated by the illumination unit (13) by using a plurality of pixels (50a) arranged in two dimensions in a row direction and in a column direction;
and
a processor apparatus (12) comprising
a controller (41) that is configured to enable a normal observation mode and a special observation mode to be selectively executed, the normal operation mode being a mode in which signal reading from the image sensor is performed in a state where the first illumination light is being emitted from the illumination unit, the special observation mode being a mode in which the first illumination light and the second illumination light are alternately emitted from the illumination unit while a turn-off period is interposed and signal reading from the image sensor (35) is performed during the turn-off period; and
an image processor (44) that is configured to generate an oxygen saturation image on the basis of a first image capture signal read from the image sensor during the turn-off period after emission of the first illumination light and a second image capture signal read from the image sensor during the turn-off period after emission of the second illumination light in the special observation mode, wherein
the controller (41) is configured to control the image sensor (39) according to a global shutter method in the special observation mode to make a second read time (T2) taken to read the second image capture signal shorter than a first read time (T1) taken to read the first image capture signal and a sum of the exposure time of the first illumination light and the first read time longer than a frame time in the normal observation mode and a sum of the exposure time of the second illumination light and the second read time shorter than the frame time in the normal observation mode.

2. The endoscope system (10) according to claim 1, wherein
the controller (41) is configured to drive the image sensor (39) in the normal observation mode in accordance with a rolling shutter method in which each row of the pixels is sequentially selected in the column direction and signal reading and resetting are performed, and to drive the image sensor in the special observation mode in accordance with a global shutter method in which the pixels (50a) are collectively reset in association with a start of emission of each of the first illumination light and the second illumination light, emission of each of the first illumination light and the second illumination light is stopped, the pixels are sequentially selected in the column direction, and signal reading is performed.

3. The endoscope system (10) according to claim 1 or 2, wherein
the plurality of pixels (50a) are divided into a plurality of types of pixel groups having different spectral sensitivities, and
the controller (41) is configured to read only signals from a pixel group of highest sensitivity to the different-absorption-wavelength light as the second image capture signal to thereby make the second read time shorter than the first read time.

4. The endoscope system (10) according to claim 1 or 2, wherein
the controller (41) is configured to increase a clock frequency of a control signal applied to the image sensor to thereby make the second read time shorter than the first read time.

5. The endoscope system (10)according to claim 1 or 2, wherein
the controller (41) is configured to drive the image sensor (39) in accordance with a pixel addition method in the column direction to thereby make the second read time shorter than the first read time.

6. The endoscope system (10) according to claim 1 or 2, wherein
the controller is configured to reduce a pixel area of the image sensor from which signals are read to thereby make the second read time shorter than the first read time.

7. The endoscope system (10) according to claim 1 or 2, wherein
the image sensor (39) is configured to include a column ADC circuit in which an ADC that converts an analog signal into a digital signal is arranged for each column of the pixels, and
the controller (41) is configured to increase a temporal change rate of a reference signal of the column ADC circuit to thereby make the second read time shorter than the first read time.

8. The endoscope system (10) according to claim 1 or 2, wherein
the controller (41) is configured to reduce a blanking interval of the image sensor to thereby make the second read time shorter than the first read time.

9. The endoscope system according to any one of claims 1 to 8, wherein
the controller (41) is configured to make an emission time of the second illumination light shorter than an emission time of the first illumination light in the special observation mode.

10. The endoscope system (10) according to any one of claims 1 to 9, wherein
the image processor (12) is configured to generate an oxygen saturation image by calculating an oxygen saturation level on the basis of the first image capture signal and the second image capture signal, to calculate a normal observation image on the basis of the first image capture signal, and to perform image processing on the normal observation image on the basis of the oxygen saturation level.

## Patentansprüche

1. Endoskopsystem (10), umfassend:
eine Beleuchtungseinheit (13), konfiguriert zum Bestrahlen eines Testkörpers mit erstem Beleuchtungslicht und zweitem Beleuchtungslicht, welches eine Spektraleigenschaft verschieden von einer Spektraleigenschaft des ersten Beleuchtungslichts hat und unterschiedliches Absorptionswellenlängen-Licht enthält, das unterschiedliche Absorptionskoeffizienten für oxigeniertes Hämoglobin und reduziertes Hämoglobin aufweist;
ein Endoskop (11) mit einem CMOS-Bildsensor (39), der konfiguriert ist zum Aufnehmen eines Bilds des mit der Beleuchtungseinheit (13) beleuchteten Testkörpers unter Verwendung mehrerer Pixel (50a), die zweidimensional in Reihenrichtung und in Spaltenrichtung angeordnet sind; und
eine Prozessorvorrichtung (12), umfassend:
eine Steuerung (41), konfiguriert zum Ermöglichen, dass ein Normalbetrachtungsmodus und ein Spezialbetrachtungsmodus selektiv ausgeführt werden, von denen der Normalbetrachtungsmodus ein Modus ist, bei dem ein Signal-Lesen von dem Bildsensor in einem Zustand ausgeführt wird, in welchem das erste Beleuchtungslicht von der Beleuchtungseinheit abgegeben wird, der Spezialbetrachtungsmodus ein Modus ist, in welchem das erste Beleuchtungslicht und das zweite Beleuchtungslicht abwechselnd von der Beleuchtungseinheit abgegeben werden, während eine Ausschaltzeitspanne dazwischen eingefügt ist, und ein Signal-Lesen von dem Bildsensor (35) während der Ausschaltzeitspanne erfolgt; und
einen Bildprozessor (44), konfiguriert zum Erzeugen eines Sauerstoffsättigungsbilds auf der Grundlage eines ersten Bildaufnahmesignals, welches von dem Bildsensor während der Ausschaltzeitspanne nach der Abgabe des ersten Beleuchtungslichts gelesen wird, und eines zweiten Bildaufnahmesignals, welches von dem Bildsensor während der Ausschaltzeitspanne nach der Abgabe des zweiten Beleuchtungslichts im Spezialbetrachtungsmodus ausgelesen wird, wobei
die Steuerung (41) konfiguriert ist zum Steuern des Bildsensors (39) gemäß einem Global-Shutter-Verfahren in dem Spezialbetrachtungsmodus, um eine zweite Lesezeit (T2) für das Lesen des zweiten Bildaufnahmesignals kürzer als eine erste Lesezeit (T1) für das Lesen des ersten Bildaufnahmesignals und eine Summe der Belichtungszeit für das erste Beleuchtungslicht und die erste Lesezeit länger zu machen als eine Einzelbildzeit in dem Normalbetrachtungsmodus, und eine Summe der Belichtungszeit für das zweite Beleuchtungslicht und die zweite Lesezeit kürzer zu machen als die Einzelbildzeit in dem Normalbetrachtungsmodus.

2. Endoskopsystem (10) nach Anspruch 1, bei dem
die Steuerung (41) konfiguriert ist zum Treiben des Bildsensors (39) in den Normalbetrachtungsmodus nach Maßgabe eines Rolling-Shutter-Verfahrens, bei dem jede Reihe von Pixeln sequentiell in der Spaltenrichtung ausgewählt wird und ein Signal-Lesen und -Zurücksetzen erfolgen, und zum Treiben des Bildsensors in dem Spezialbetrachtungsmodus nach Maßgabe eines Global-Shutter-Verfahrens, in welchem die Pixel (50a) kollektiv in Verbindung mit einem Start der Emission sowohl des ersten Beleuchtungslichts als auch des zweiten Beleuchtungslichts zurückgesetzt werden, die Abgabe des ersten Beleuchtungslichts und auch des zweiten Beleuchtungslichts angehalten wird, die Pixel sequentiell in der Spaltenrichtung ausgewählt werden, und das Signal-Lesen ausgeführt wird.

3. Endoskopsystem (10) nach Anspruch 1 oder 2, bei dem die mehreren Pixel (50a) unterteilt sind in mehrere Typen von Pixelgruppen unterschiedlicher Spektralempfindlichkeiten, und
die Steuerung (41) konfiguriert ist zum Lesen von lediglich Signalen einer Pixelgruppe höchster Empfindlichkeit für das Licht unterschiedlicher Absorptionswellenlänge als das zweite Bildaufnahmesignal, um dadurch die zweite Lesezeit kürzer zu machen als die zweite Lesezeit.

4. Endoskopsystem (10) nach Anspruch 1 oder 2, bei dem die Steuerung (41) konfiguriert ist zum Erhöhen einer Taktfrequenz eines an den Bildsensor angelegten Steuersignals, um dadurch die zweite Lesezeit kürzer zu machen als die erste Lesezeit.

5. Endoskopsystem (10) nach Anspruch 1 oder 2, bei dem die Steuerung (41) konfiguriert ist zum Treiben des Bildsensors (39) nach Maßgabe eines Pixeladditionsverfahrens in Spaltenrichtung, um dadurch die zweite Lesezeit kürzer zu machen als die erste Lesezeit.

6. Endoskopsystem (10) nach Anspruch 1 oder 2, bei dem die Steuerung konfiguriert ist zum Reduzieren einer Pixelfläche des Bildsensors, aus der Signale gelesen werden, um dadurch die zweite Lesezeit kürzer zu machen als die erste Lesezeit.

7. Endoskopsystem (10) nach Anspruch 1 oder 2, bei dem der Bildsensor eine Spalten-ADC-Schaltung enthält, in der für jede Pixelspalte ein ADC (Analog-Digital-Wandler) angeordnet ist, der ein Analogsignal in ein Digitalsignal umwandelt, und
die Steuerung (41) konfiguriert ist zum Erhöhen einer zeitlichen Änderungsrate eines Referenzsignals der Spalten-ADC-Schaltung, um dadurch die zweite Lesezeit kürzer zu machen als die erste Lesezeit.

8. Endoskopsystem (10) nach Anspruch 1 oder 2, bei dem
die Steuerung (41) konfiguriert ist zum Reduzieren eines Austastintervalls des Bildsensors, um dadurch die zweite Lesezeit kürzer als die erste Lesezeit zu machen.

9. Endoskopsystem nach einem der Ansprüche 1 bis 8, bei dem
die Steuerung (41) konfiguriert ist, um eine Abgabezeit für das zweite Beleuchtungslicht gegenüber der Abgabezeit des ersten Beleuchtungslichts in dem Spezialbetrachtungsmodus zu verkürzen.

10. Endoskopsystem (10) nach einem der Ansprüche 1 bis 9, bei dem
der Bildprozessor (12) konfiguriert ist zum Erzeugen eines Sauerstoffsättigungsbilds durch Berechnen eines Sauerstoffsättigungswerts auf der Grundlage des ersten Bildaufnahmesignals und des zweiten Bildaufnahmesignals, um ein Normalbetrachtungsbild zu berechnen auf der Grundlage des ersten Bildaufnahmesignals und um eine Bildverarbeitung an dem Normalbetrachtungsbild auf der Grundlage des Sauerstoffsättigungswerts vorzunehmen.

## Revendications

1. Système d'endoscope (10), comprenant :
une unité d'éclairage (13), laquelle est configurée pour irradier un corps d'essai avec une première lumière d'éclairage et une seconde lumière d'éclairage présentant une propriété spectrale différente d'une propriété spectrale de la première lumière d'éclairage et incluant une lumière de longueur d'onde d'absorption différente, laquelle présente des coefficients d'absorption différents pour l'oxyhémoglobine et la désoxyhémoglobine ;
un endoscope (11) incluant un capteur d'image CMOS (39), lequel est configuré pour capturer une image du corps d'essai éclairé par l'unité d'éclairage (13) à l'aide d'une pluralité de pixels (50a) agencés de manière bidimensionnelle dans une direction de rangée et une direction de colonne, et
un appareil à processeur (12), comprenant
un contrôleur (41), lequel est configuré pour permettre l'exécution de manière sélective d'un mode d'observation normal et d'un mode d'observation spécial, le mode de fonctionnement normal étant un mode où une lecture de signal à partir du capteur d'image est réalisée, dans un état où la première lumière d'éclairage est en cours d'émission à partir de l'unité d'éclairage, le mode d'observation spécial étant un mode où la première lumière d'éclairage et la seconde lumière d'éclairage sont émises par alternance à partir de l'unité d'éclairage tandis qu'une période hors tension est intercalée et que la lecture de signal à partir du capteur d'image (35) est réalisée durant la période hors tension, et
un processeur d'image (44), lequel est configuré pour générer une image de saturation en oxygène, sur la base d'un premier signal de capture d'image lu à partir du capteur d'image durant la période hors tension après émission de la première lumière d'éclairage et d'un second signal de capture d'image lu à partir du capteur d'image durant la période hors tension après émission de la seconde lumière d'éclairage dans le mode d'observation spécial, dans lequel
le contrôleur (41) est configuré pour commander le capteur d'image (39) conformément à un procédé d'obturateur global dans le mode d'observation spécial afin de rendre un second temps de lecture (T2) pris pour lire le second signal de capture d'image, plus court qu'un premier temps de lecture (T1) pris pour lire le premier signal de capture d'image, et une somme du temps d'exposition de la première lumière d'éclairage avec le premier temps de lecture plus longue qu'un temps d'image dans le mode d'observation normal, et une somme du temps d'exposition de la seconde lumière d'éclairage avec le second temps de lecture plus courte que le temps d'image dans le mode d'observation normal.

2. Système d'endoscope (10) selon la revendication 1, dans lequel
le contrôleur (41) est configuré pour exciter le capteur d'image (39) dans le mode d'observation normal conformément à un procédé d'obturateur déroulant, où chaque rangée des pixels est sélectionnée de manière séquentielle dans la direction de colonne, et où une lecture de signal et une réinitialisation sont réalisées, et pour exciter le capteur d'image dans le mode d'observation spécial conformément à un procédé d'obturateur global, où les pixels (50a) sont réinitialisés de manière collective en association à un début d'émission de chaque lumière parmi la première lumière d'éclairage et la seconde lumière d'éclairage, l'émission de chaque lumière parmi la première lumière d'éclairage et la seconde lumière d'éclairage est arrêtée, et les pixels sont sélectionnés de manière séquentielle dans la direction de colonne, et une lecture de signal est réalisée.

3. Système d'endoscope (10) selon la revendication 1 ou 2, dans lequel
la pluralité de pixels (50a) sont divisés en une pluralité de types de groupes de pixels présentant des sensibilités spectrales différentes, et
le contrôleur (41) est configuré pour ne lire que des signaux provenant d'un groupe de pixels présentant la sensibilité la plus élevée à la lumière de longueur d'onde d'absorption différente comme second signal de capture d'image, afin de rendre ainsi le second temps de lecture plus court que le premier temps de lecture.

4. Système d'endoscope (10) selon la revendication 1 ou 2, dans lequel
le contrôleur (41) est configuré pour augmenter une fréquence d'horloge d'un signal de commande appliqué au capteur d'image, afin de rendre ainsi le second temps de lecture plus court que le premier temps de lecture.

5. Système d'endoscope (10) selon la revendication 1 ou 2, dans lequel
le contrôleur (41) est configuré pour exciter le capteur d'image (39) conformément à un procédé d'addition de pixels dans la direction de colonne, afin de rendre ainsi le second temps de lecture plus court que le premier temps de lecture.

6. Système d'endoscope (10) selon la revendication 1 ou 2, dans lequel
le contrôleur est configuré pour réduire une zone de pixels du capteur d'image d'où des signaux sont lus, afin de rendre ainsi le second temps de lecture plus court que le premier temps de lecture.

7. Système d'endoscope (10) selon la revendication 1 ou 2, dans lequel
le capteur d'image (39) est configuré pour inclure un circuit de conversion analogique-numérique (ADC) de colonne, où un ADC convertissant un signal analogique en un signal numérique est agencé pour chaque colonne des pixels, et
le contrôleur (41) est configuré pour augmenter un taux de changement temporel d'un signal de référence du circuit ADC de colonne, afin de rendre ainsi le second temps de lecture plus court que le premier temps de lecture.

8. Système d'endoscope (10) selon la revendication 1 ou 2, dans lequel
le contrôleur (41) est configuré pour réduire un intervalle de suppression du capteur d'image, afin de rendre ainsi le second temps de lecture plus court que le premier temps de lecture.

9. Système d'endoscope selon l'une quelconque des revendications 1 à 8, dans lequel
le contrôleur (41) est configuré pour rendre un temps d'émission de la seconde lumière d'éclairage plus court qu'un temps d'émission de la première lumière d'éclairage dans le mode d'observation spécial.

10. Système d'endoscope (10) selon l'une quelconque des revendications 1 à 9, dans lequel
le processeur d'images (12) est configuré pour générer une image de saturation en oxygène, en calculant un niveau de saturation en oxygène sur la base du premier signal de capture d'image et du second signal de capture d'image, afin de calculer une image d'observation normale sur la base du premier signal de capture d'image, et pour réaliser un traitement d'images sur l'image d'observation normale sur la base du niveau de saturation en oxygène.
